# EUROPEAN PATENT APPLICATION

(11) **EP 2 589 387 A1**
(43) Date of publication of application: **08.05.2013**
(21) Application number: 11008798.8
(22) Date of filing: 04.11.2011
(51) Int. Cl.: A61K 31/715, A61K 31/739, A61P 35/04, A61K 9/00

(54) **Use of a teichoic acid for the treatment of malignant liquor cerebrospinalis in the brain**

(71) Applicant: Lunamed AG, 7000 Chur (CH)
(72) Inventor: Truog, Peter, 7000 Chur (CH)
(74) Representative: Peters, Hajo

(57) **Abstract**

The present invention refers to the use of a teichoic/lipoteichoic acid, especially of LTA-T, for the treatment of malignant liquor cerebrospinalis. Thus, the invention relates to the treatment of malignant liquor cerebrospinalis, wherein the liquor cerebrospinalis carries malignant cells. The present invention also relates to the use of teichoic or lipoteichoic acid in the manufacture of a medicament for treating malignant liquor cerebrospinalis. The present invention also relates to a kit comprising a pharmaceutical composition comprising a teichoic or lipoteichoic acid and instructions indicating that the composition is for use in malignant liquor cerebrospinalis. In addition, the present invention relates to a method of treating malignant liquor cerebrospinalis comprising administration of teichoic acid/lipoteichoic acid to a subject.

## Description

### Field of the invention

The present invention refers to the use of a teichoic/lipoteichoic acid, especially of LTA-T, for the treatment of malignant liquor cerebrospinalis. Thus, the invention relates to the treatment of malignant liquor cerebrospinalis, wherein the liquor cerebrospinalis carries malignant cells. The present invention also relates to the use of teichoic or lipoteichoic acid in the manufacture of a medicament for treating malignant liquor cerebrospinalis. The present invention also relates to a kit comprising a pharmaceutical composition comprising a teichoic or lipoteichoic acid and instructions indicating that the composition is for use in malignant liquor cerebrospinalis. In addition, the present invention relates to a method of treating malignant liquor cerebrospinalis comprising administration of teichoic acid/lipoteichoic acid to a subject.

### Background of the invention

Malignant Liquor cerebrospinalis or Liquor cerebrospinalis carrying malignant cells is a widely occurring medical complication in which the liquor cerebrospinalis in the CNS is containing malignant cells. These malignant cells are either generated by tumours of the brain or by metastasis in which tumours from other regions of the body invade the brain or liquor cerebrospinalis. The latter case is also known as menigeal carcinosis, the transfection of the meninges with metastasis. 2 to 34 % of these malignant tumours occur due to primary brain cancer, whereas solid tumours of mama-carcinoma, lung carcinoma and melanoma are with 52-72% the most common causes of neoplastic meningitis causing malignant liquor cerebrospinalis due to metastasis, followed by hematologic neoplasm with 11 % to 36% occurrence. Spread of Cancer to the meninges is called malignant meninges.

Focussing on primary brain cancer one has to distinguish between occurrence in adult and children. In children, the primary brain cancer involves either oligodendroglioma or astrocytoma. Oligodendroglioma are reacting quite well to radiotherapy or chemotherapy while there is no proper therapy for astrocytoma. In adults the main part of primary brain cancer involves glioblastoma with no therapy being available.

Thus, there is a clear need for a new, effective way of treating malignant liquor cerebrospinalis, preferably also having reduced side effects.

The applicant has now found-out that teichoic acids, especially lipoteichoic acids, like LTA-T, seem to show a positive response in malignant liquor cerebrospinalis. Lipoteichoic acid as LTA-T is generally well tolerated and no specific side effects are observed. A response of malignant liquor cerebrospinalis to LTA-T is expected to show in a large proportion of the patients treated, a complete disappearance of the malignant tumour cells within days. The effect seems present at even one or very few single treatments and/or at doses as low as 250 µg of lipoteichoic acid. In summary, from first results and conclusions drawn it seems clear that teichoic/lipoteichoic acids seem adequate to treat malignant liquor cerebrospinalis, thus solving the basic problem.

Thus, the present invention relates to a teichoic acid for use in the treatment of malignant liquor cerebrospinalis.

WO2009/30929 A1 does disclose the use of Lipoteichoic Acid T (LTA-T) in treating pleural effusion or pneumothorax. Even though, there could be seen a loose relation between the forming of the fluids in pleural effusion and the presence and occurrence of malignant liquor cerebrospinalis, the basic teaching of WO2009/030929 A1 is actually quite opposed to the basic problem of the current invention, even teaching away from the inventive solution. Opposed to the current invention, WO2009/030929 A1 associates the treatment of the pleural effusion fully on an alleged activity of LTA-T as a pleurodesis agent with pleurodesis being the adherence of the lung to the chest wall (WO2009/30929, page 1, lines 8, and 13-14; page 2, line 5 to 11 or especially page 2, line 30 to page 3, line 5). Especially, this last part of WO2009/30929 strongly reflects the teaching of this piece of art, which states that by the inflammation induced by LTA-T, adherence of the pleural membranes by fibrin is triggered, thus leading to the effect that pleural effusion is stopped. This is naturally not applying at all to the situation in the brain and a malignant liquor cerebrospinalis, just the opposite. There are no (pleural) membranes whose adherence to each other would be possibly beneficial to treat malignant liquor cerebrospinalis in the brain. In addition, in a situation, with malignant liquor cerebrospinalis, the last thing that is wanted is a further pro-inflammatory reaction in the brain by a lipoteichoic acid like LTA-T causing a form of meningitis. Thus, reading WO2009/30929 and taking its teaching as a basis, the expert would be clearly led away from applying the treatment with LTA also to malignant liquor cerebrospinalis as the taught acitivities of LTA-T would be very counterproductive for any solution of the problem of malignant liquor cerebrospinalis treatment.

A very similar situation arises when taking a look at the article of Rahman et al.; "Use of lipoteichoic acid-T for pleurodesis in malignant pleural effusion: a phase I toxicity and dose-escalation study", Lancet Ocol. (2008). As already mentioned in the title and further found throughout the article, the teaching of this Lancet publication was again only focussed on pleurodesis. Already the first paragraph of the abstract is fully clear in that, postulating that administering lipoteichoic acid (LTA-T) would cause an adherence of the pleural membranes (pleurodesis), probably mediated by inflammation. This is then seen and described as the mechanism underlying the successful treatment of patients suffering from pleural effusion. As said above, this would not apply to malignant liquor cerebrospinalis as there are no (pleural) membranes in the brain whose adherence could potentially be beneficial and pro-inflammatory reaction would not seem positive at all in the brain. Thus again, this article and its teaching would have lead an expert - charged with finding a solution to the problem of treating malignant liquor cerebrospinalis - away from the solution offered by this application: treatment of malignant tumors with teichoic/lipoteichoic acid.

The applicant was thus highly surprised to find out that opposed to the expectations induced by the teaching of WO2009/30929 and Rahman et al. (2008), teichoic/lipoteichoic acid seems able to remove malignant cells from the liquor cerebrospinalis, an effect that cannot be explained by and is even in contradiction to the teaching of WO2009/30929. The applicant is even considering that the alleged pleurodesis taught in WO2009/30929 as the underlying mechanism of action of LTA-T in the treatment of pleural effusion might not be the right explanation for the effect.

"Malignant liquor cerebrospinalis" is defined herein as liquor cerebrospinalis containing malignant cells. Liquor cerebrospinalis is found in the Central Nervous System. The malignant cancer cells found in the malignant liquor cerebrospinalis are stemming from cancer either primary brain cancer or from metastasis invading the brain/liquor from tumours outside the brain, like solid mama-carcinoma, or lung carcinoma or melanoma. The fluid of the "malignant liquor cerebrospinalis" does contain thus malignant tumor cells.

"Treatment of malignant liquor cerebrospinalis" is defined herein as treatment of the "malignant liquor cerebrospinalis", especially by injection of the teichoic/lipoteichoic acid (LTA-T) into the liquor cerebrospinalis fluid of the malignant liquor cerebrospinalis in the brain or CNS (intrathecally). Treatment thus results and can be measured on disappearance of malignant tumour cells from the liquid (mostly within days of the first treatment).

"Teichoic acids" as defined herein is an inclusive term including the a) lipoteichoic acids (LTA) and b) the wall teichoic acids (WTA) form a major part of the cell wall of gram-positive bacteria.

Literature - both scientific and patent - on LTA has become abundant in the last years with a selected few of them mentioned below spanning themes like:
- Mode of Action:
   o Bunk et al., J. Immunol. (2010) 185(6), 3708-3717 ;
   o Ding, Peijie, Huazhong Keiji,(2009), 38(6), 812-814 (abstract) ;
   o Shiratsuchi et al., Immunology (2010), 129(2), 268-277;
   o Tang et al., Biochemical Pharmacology (2010), 79(11), 1648-1657;
   o Keller et al., Innate Immunity (2011), 17(1), 29-34 ;
- Inflammation:
   o van Zoelen et al., Molec. Immunol. (2011), 48(12-13), 1468-1476;
   o Long et al., PLoS One (2009) 4(5),
- Synthesis/Production Processes:
   o Rahman et al., Trends in Microbiol. (2009), 17(6), 219-215;
   o McCormick et al., Microbiology (2011), 157(8), 2248-2256;
   o Qiao et al., Bioorganic & Medicinal Chemistry (2010), 18(11),3696-3702;
   o Pedersen et al. Angewandte Chemie (2010), 49(14), 2585-2590 (see below)
   o Schmidt et al., Organic & Biomolecular Chemsitry (2011), 9(7),2040-2052;
   o Pedersen, Microbial Glycobiology; Edit.. Moran A.-P. (2009), 455-476
- Analytics:
   o Sanchez-Carballo et al., Carbohydrate Res. (2010), 345(4), 538-542;
   o Jang et al. Biochem. Biophys. Res. Com. (2011), 407(4), 823-830.

A very preferred aspect and embodiment of the invention refers to the teichoic acid, as defined above, for use in the treatment of malignant liquor cerebrospinalis, wherein the teichoic acid is a lipoteichoic acid. There are many lipoteichoic acids known which form a major part of the cell wall of gram-positive bacteria.

The known lipoteichoic acids include synthetic, semisynthetic and naturally occurring lipoteichioic acids. A very broad introduction on the synthesis of lipoteichoic acids can be found in Pedersen, Christian Marcus; Schmidt, Richard R. (Edited by Moran, Anthony P), Microbial Glycobiology (2009), 455-476 with all lipoteichoic acids described therein herewith included by reference n this application as examples of the lipoteichoic acids to be used.. A further overview of the lipoteichoic acids ― divided in Types I to IV - can be found in Greenberg et al., Infect Immun. 1996 Aug;64(8):3318-25. All lipoteichoic acids Type I, Type II, Type III and Type IV as well as all those described therein (like e.g. in tables 1 or 2) are herewith included by reference in this application as examples of the lipoteichoic acids for use in the treatment of malignant liquor cerebrospinalis.

The (general) lipoteichoic acids as defined in this application are comprising:
a) a linear, hydrophilic 1,3-connected glycero-phophate chain - optionally substituted with D-alanine or other molecules -,
b) the gylcerophosophate chain being covalently bound through a phosphodiester to
c) a glycolipid, preferably a glyceroglycolipid.

Lipoteichoic acids falling under this definition according to this invention are called "(general) lipoteichoic acids" already before and hereinafter.

The glycolipid acts as an anchor in the cell membrane. The glycolipids are by definition of the invention lipids of the membrane in which one or more mono- or oligosaccharids are bound via a glycosidic bond to a lipid molecule. The lipid molecule is consisting of fatty acids being bound by an ester bond to a glycerol or by an amid bond to a sphingosine.

Preferably the glycolipid is a glyceroglycolipid in which one or more mono- or oligosaccharids are bound via a glycosidic bond to a lipid molecule consisting of fatty acids being bound by an ester bond to the glycerol. Preferably the glyceroglycolipid comprises a diacylglycerol with two fatty acid chains of 10 to 20 carbon atoms each being bound by an ester bond to the glycerol. Preferably the fatty acid chains are linear or branched and non-substituted and/or have a length of 10 to 20 carbon atoms each, most preferably 12, 14, 16 or 18 carbon atoms.

Most (general) lipoteichoic acids would be covered by the general formula I and its substituents as described above and directly below and some preferred embodiments are described below, but some are unusual. These unusual include the lipoteichoic acid from *Streptococcus pneumoniae* (see below) whose synthesis was recently described by Pedersen et al., in Angew. Chem. (2010), 122, 1 - 7. 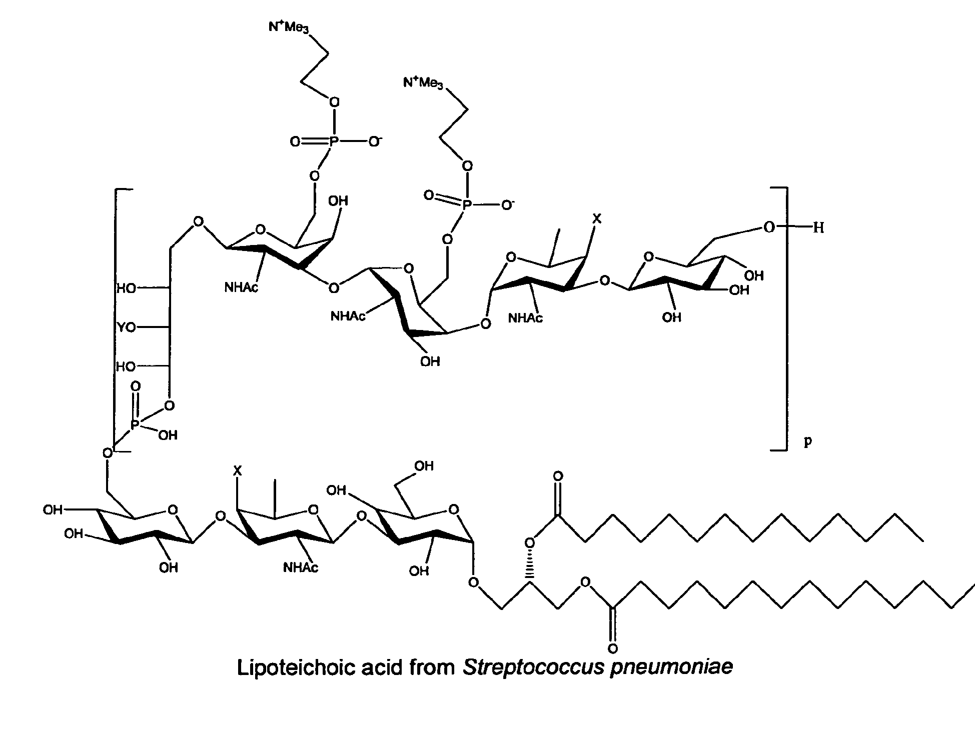 wherein
in one case X is NH₃⁺, or NHAc; Y is H, D-Ala, or α-GalNAc; and p is up to 8;
in the other case X is NH₃⁺; Y is H; and p is 1.

In one preferred embodiment of the invention (referring to a lipoteichoic acid) the glycerophosphate derivative used according to the invention is a lipoteichoic acid according to general formula I 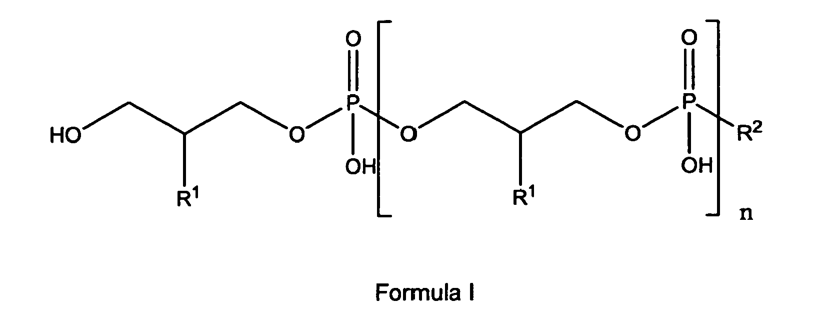 wherein
the mean value of n is 8 to 40,
R¹ is either OH or O-D-alanyl and R² is either 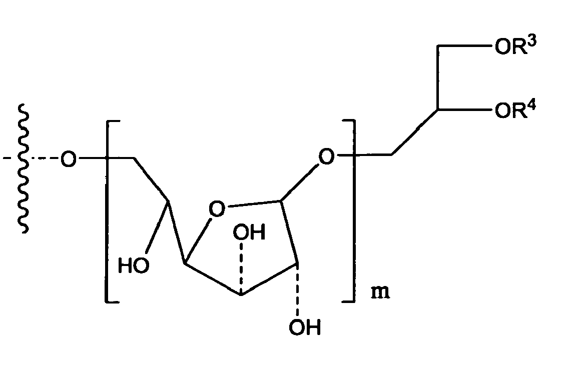 or its tautomeric equivalent 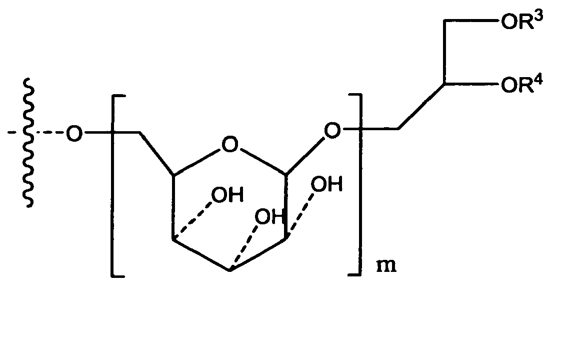 with R³ and R4 independently from one another being residues of saturated or unsaturated, unsubstituted fatty acids with 10 to 20 carbon atoms and m being selected from 1 to 3,
with the compounds of formula I being present either in free form or as physiologically acceptable salt; in form of any of their structural isomers including mixtures thereof, preferably in pure enantiomeric or diastereomeric form or any mixture thereof including racemic mixtures.

In one further even more preferred embodiment of the invention (referring to a lipoteichoic acid) the glycerophosphate derivative used according to the invention is a lipoteichoic acid according to general formula I 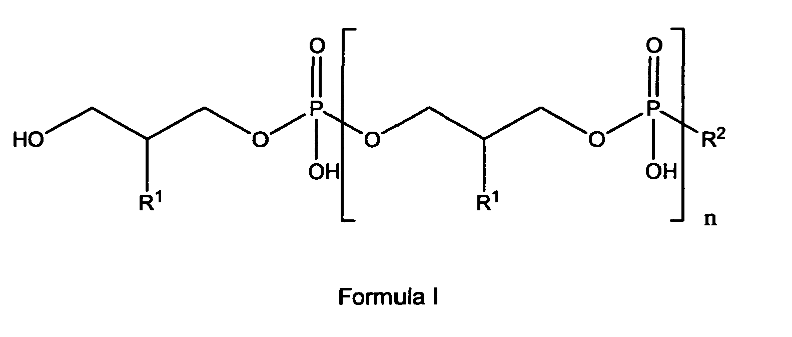 wherein
the mean value of n is 9,
R¹ is either OH or O-D-Alanyl and
R² is 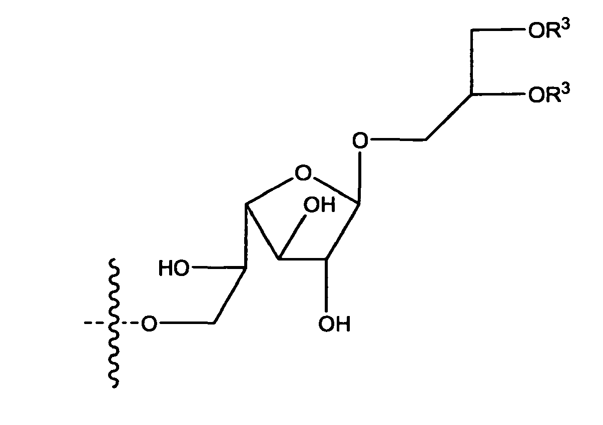 with R³ being residues of saturated or unsaturated fatty acids with 12, 14, 16 or 18 carbon atoms,
with the compounds of formula I being present either in free form or as physiologically acceptable salt; in form of any of their structural isomers including mixtures thereof, preferably in pure enantiomeric or diastereomeric form or any mixture thereof including racemic mixtures. These compounds (lipoteicoic acids) defined directly above as well as those described by general formula III, IIIa, IIIb, or IIIc and their defined radicals below (as well as any lipoteichoic acid to be isolated from the Streptococcus sp. PT strain DSM 8747) are defined in this invention as "lipoteichoic acid of the invention" or "lipoteichoic acid used according to the invention".
In a further embodiment the invention relates to a purified lipoteichoic acid isolatable from the Streptococcus sp. PT strain DSM 8747, preferably containing a beta-galactofuranosyl(1-3)glycerol-di-ester moiety, for use in the treatment of malignant liquor cerebrospinalis.

In one preferred embodiment (referring to a lipoteichoic acid) the lipoteichoic acid used according to the invention is a compound according to any of general formulas II, IIa, IIb, or Ilc 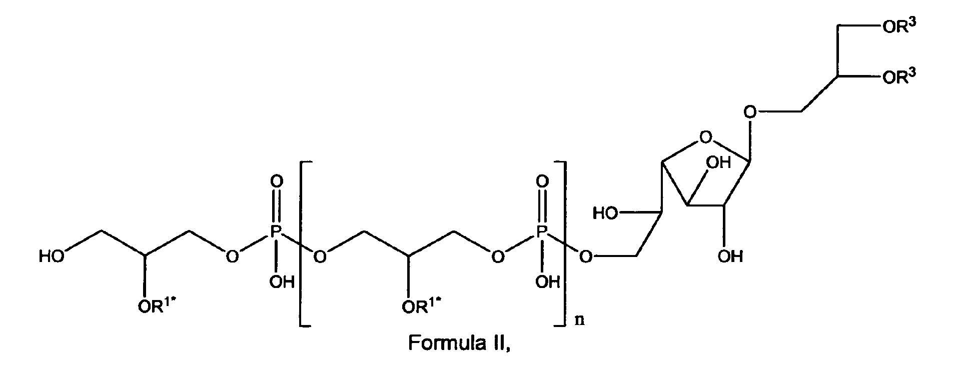 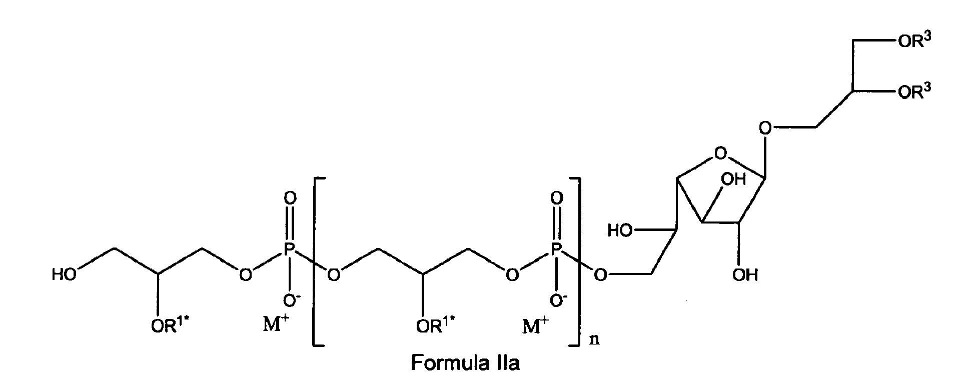 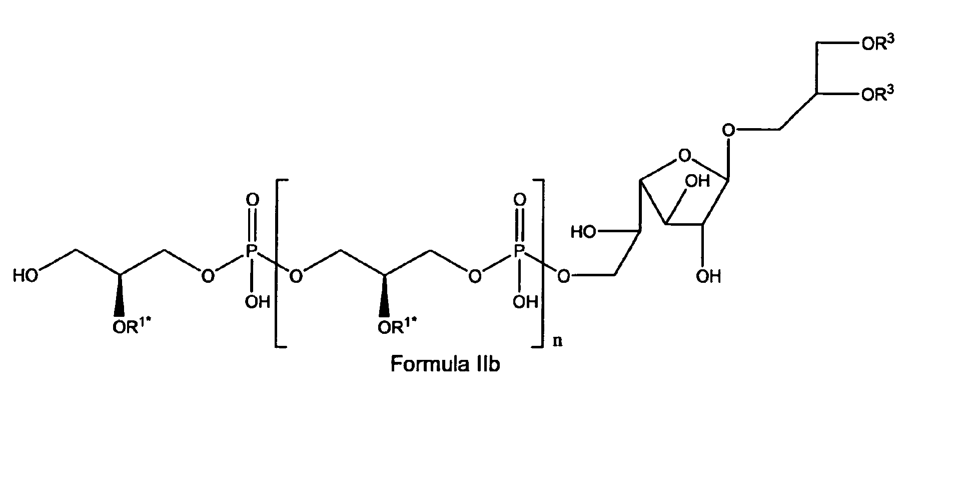 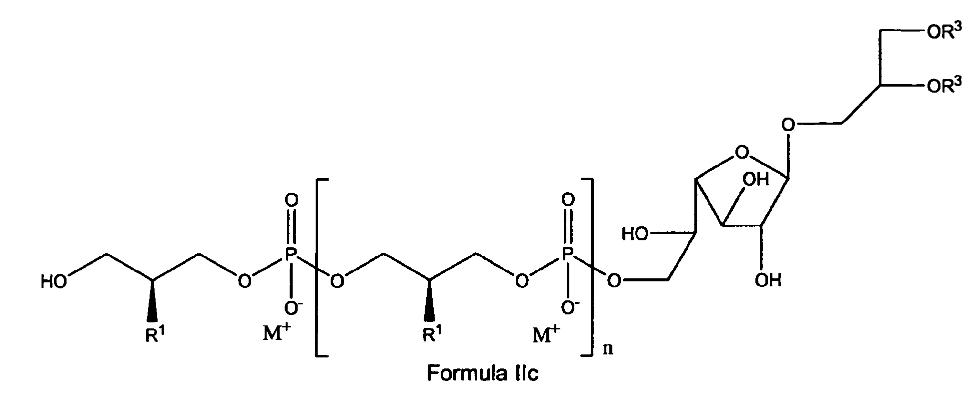 with
n having a mean value of 9, preferably being 6 to 12 while having a mean value of 9,
R^{1*} being either H or D-alanyl,
R³ being residues of saturated or unsaturated fatty acids with 12, 14, 16 or 18 carbon atoms, and
M⁺ being selected from positively charged ions, such as alkali metal ions or positively charged primary, secondary, tertiary or quaternary ammonium ions, preferably M⁺ being a sodium ion.

In one preferred embodiment (referring to a lipoteichoic acid) the lipoteichoic acid used according to the invention is a purified lipoteichoic acid (hereinafter called LTA-T) isolated from bacteria of the genus *streptococcus,* preferably from *streptococcus sp.,* most preferably from *streptococcus sp.* (DSM 8747).

Lipoteichoic acids - especially the lipoteichoic acid (LTA-T) - are described in the PCT-application WO 96/23896 together with its antitumor cholesterol-lowering activity and ways of isolating the LTA-T. The whole disclosure of WO06/23896 is included in this application by way of reference.

Thus, in one preferred embodiment of the teichoic acid used according to the invention, the lipoteichoic acid is LTA-T, a purified lipoteichoic acid isolatable from the Streptococcus sp. PT strain DSM 8747.

In one further embodiment (referring to a lipoteichoic acid) the lipoteichoic acid used according to the invention being a compound according to general formula I is a lipoteichoic acid of *staphylococcus aureus* (below): 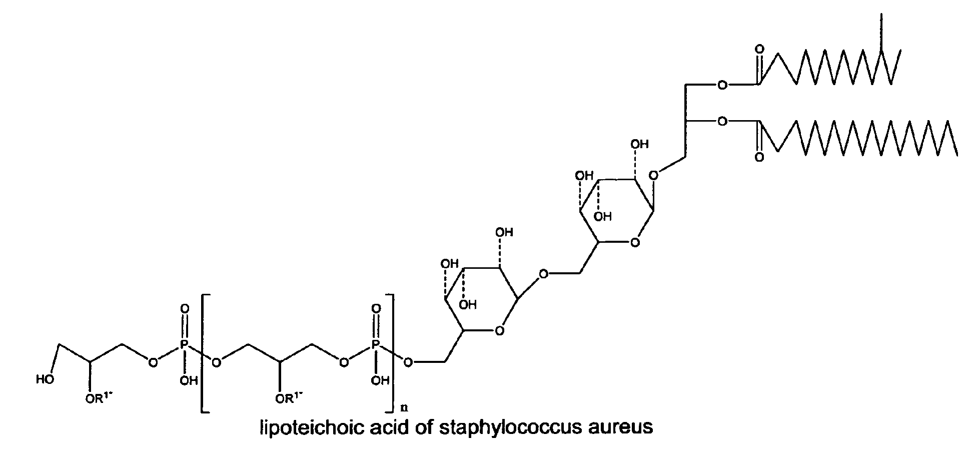 , wherein X is H or D-ala and the mean value of "n" is 16 to 40.

In a further preferred embodiment of the teichoic acid used according to the invention, the teichoic or lipoteichoic acid or its physiological acceptable salt is prepared for the use in the treatment of malignant liquor cerebrospinalis in form of an injectable fluid.

In a further preferred embodiment of the teichoic acid used according to the invention, the teichoic or lipoteichoic acid or its physiological acceptable salt is used by injection of the teichoic or lipoteichoic acid into the fluid of the malignant liquor cerebrospinalis.

In a further preferred embodiment of the teichoic acid used according to the invention,the teichoic or lipoteichoic acid or its physiological acceptable salt is used by applying 100 µg to 500 µg, preferably 250µg, of teichoic or lipoteichoic acid or its physiological acceptable salts, preferably by injection of 100 µg to 500 µg, preferably 250 µg, of the teichoic or lipoteichoic acid or its physiological acceptable salts.

A further aspect of the invention relates to the use of a teichoic or lipoteichoic acid or its physiological acceptable salts as defined above in the manufacture of a medicament for the treatment of malignant liquor cerebrospinalis.

Another further aspect of the invention relates to a pharmaceutical composition comprising teichoic or lipoteichoic acid or its physiological acceptable salts as defined in above and optionally at least one physiologically acceptable excipient for use in the treatment of malignant liquor cerebrospinalis. Preferably, this pharmaceutical composition would have the form of an injectable fluid, preferably an injectable fluid comprising injectable liquid carriers, such as water or suitable alcohols, and/or pharmaceutical excipients for injection, such as stabilizing agents, solubilizing agents, and buffers. Further preferably, this pharmaceutical composition would be comprising 10 to 1000 µg, or 100 µg to 500 µg, preferably about 250 µg of teichoic/lipoteichoic acid or its physiological acceptable salts, preferably being in form of an injectable fluid comprising 10 to 1000 µg, or 100 µg to 500 µg, preferably about 250 µg of teichoic or lipoteichoic acid or its physiological acceptable salts, preferably in form of an injectable fluid to be injected by intraperitoneal route.

Another further aspect of the invention relates to a kit comprising a pharmaceutical composition comprising teichoic or lipoteichoic acid or its physiological acceptable salts as defined above and instructions indicating that the composition is for use in malignant liquor cerebrospinalis.

Another further aspect of the invention relates to a method of treating malignant liquor cerebrospinalis comprising administration of teichoic acid/lipoteichoic acid or its physiological acceptable salts as defined above to a subject in need thereof.

For the medicament or pharmaceutical formulation according to the present invention or for a medicament or pharmaceutical formulation used according to the invention the medicament or pharmaceutical formulation can be of any form suitable for the application to humans and/or animals, preferably humans including infants, children and adults and can be produced by standard procedures known to those skilled in the art. The medicament or pharmaceutical formulation can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modem Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the route of administration.

Most preferably though, the medicament or pharmaceutical formulation of the present invention may take the form of an injectable formulation. Thus, it can for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments or pharmaceutical formulation may for example be injected intraperitoneally.

The daily dosage for humans and animals may vary depending on factors that have their basis in the respective species or other factors, such as age, sex, weight or degree of illness and so forth. The daily dosage for humans preferably is in the range of 10 µg to 1000 µg, preferably 100 µg to 500 µg, preferably about 250 µg of teichoic/lipoteichoic acid or its physiological acceptable salts to be administered during one or several intakes/injections per day.

## Claims

1. A teichoic acid for use in the treatment of malignant liquor cerebrospinalis.

2. The teichoic acid used according to claim 1, wherein the teichoic acid is a lipoteichoic acid.

3. The teichoic acid used according to claim 2, wherein the lipoteichoic acid comprises
a) a linear, hydrophilic 1,3-connected glycero-phophate chain - optionally substituted with D-alanine or other molecules -,
b) the gylcerophosophate chain being covalently bound through a phosphodiester to
c) a glycolipid, preferably a glyceroglycolipid.

4. The teichoic acid used according to claim 2, wherein the lipoteichoic acid is a
lipoteichoic acid according to general formula I 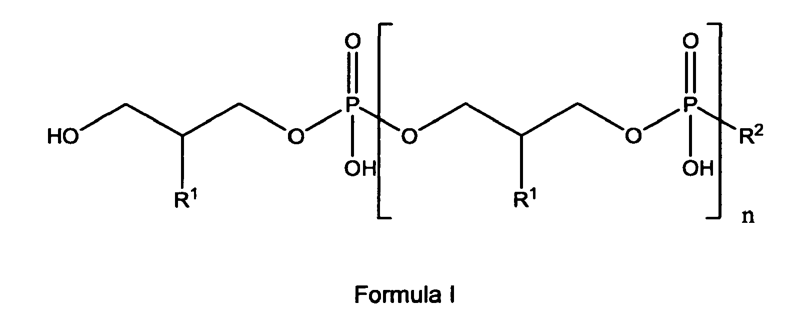 wherein
the mean value of n is 8 to 40, R¹ is either OH or O-D-alanyl and R² is
either 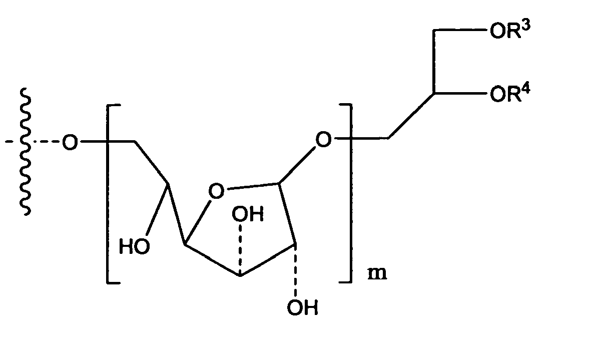 or its tautomeric equivalent 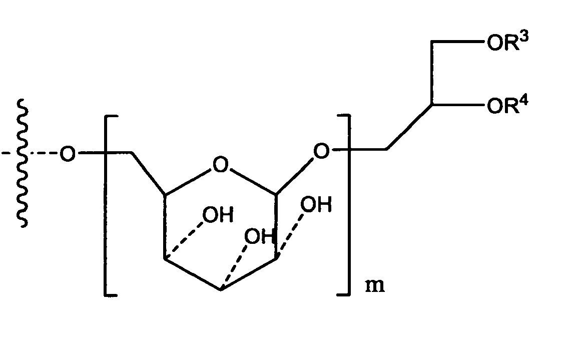 with R³ and R⁴ independently from one another being residues of saturated or unsaturated, unsubstituted fatty acids with 10 to 20 carbon atoms and m being selected from 1 to 3,
with the compounds of formula I being present either in free form or as physiologically acceptable salt; in form of any of their structural isomers including mixtures thereof, preferably in pure enantiomeric or diastereomeric form or any mixture thereof including racemic mixtures.

5. The teichoic acid used according to any one of claims 2 to 4, wherein the lipoteichoic acid is a lipoteichoic acid according to general formula I 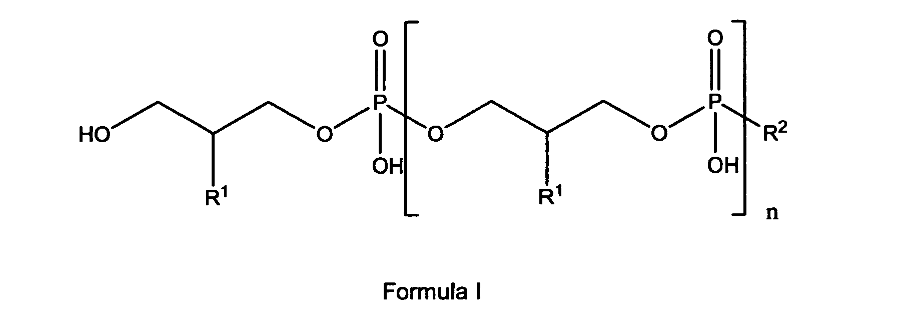 wherein
the mean value of n is 9,
R¹ is either OH or O-D-Alanyl and R² is 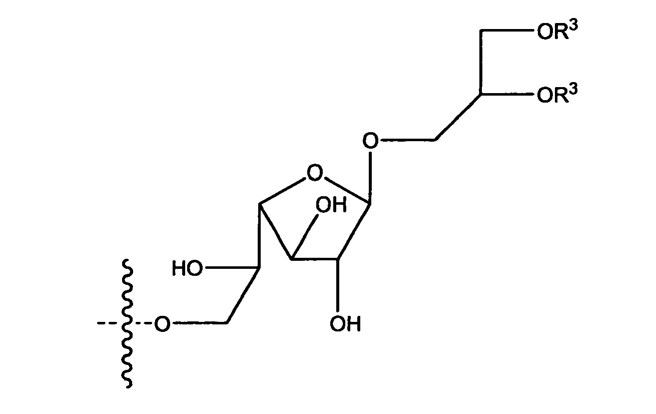 with R³ being residues of saturated or unsaturated fatty acids with 12, 14, 16 or 18 carbon atoms,
with the compounds of formula I being present either in free form or as physiologically acceptable salt; in form of any of their structural isomers including mixtures thereof, preferably in pure enantiomeric or diastereomeric form or any mixture thereof including racemic mixtures.

6. The teichoic acid used according to any one of claims 2 to 5, wherein the lipoteichoic acid is a compound according to any of general formulas II, IIa, IIb, or IIc 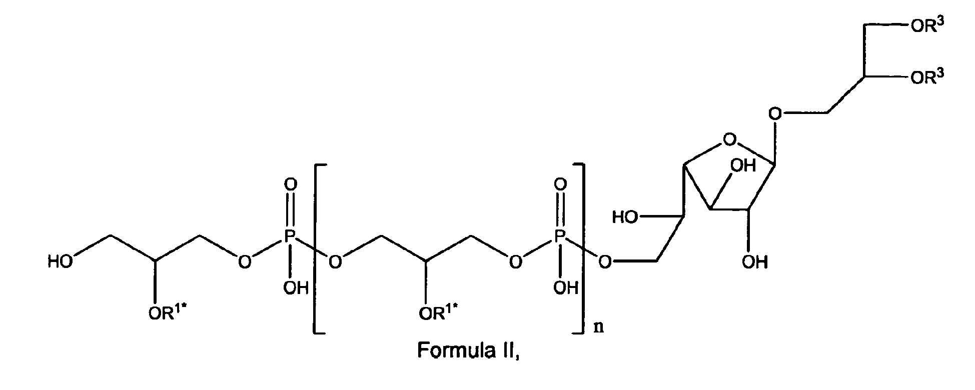 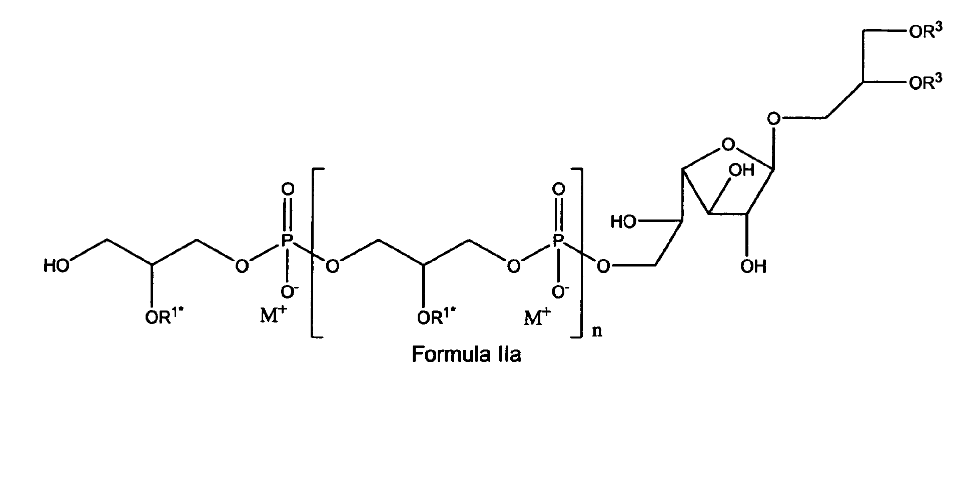 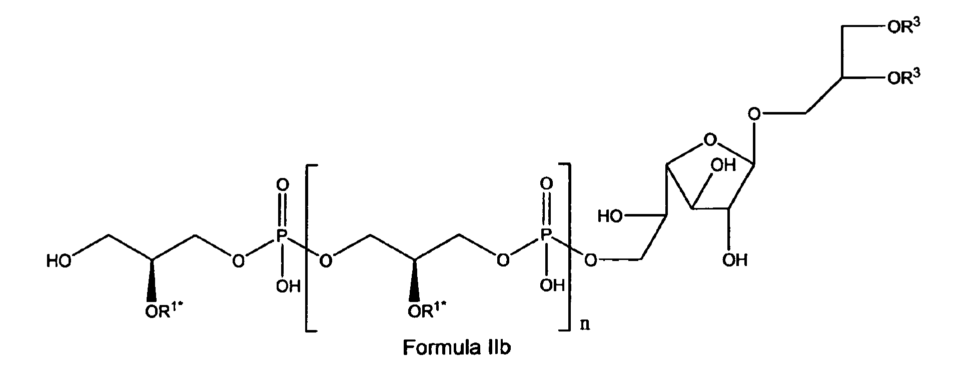 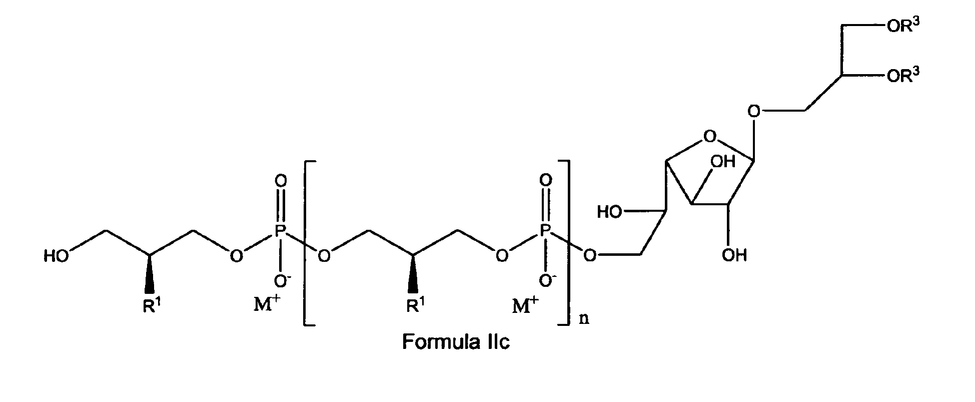 with
n having a mean value of 9, preferably being 6 to 12 while having a mean value of 9,
R^{1*} being either H or D-alanyl,
R³ being residues of saturated or unsaturated fatty acids with 12, 14, 16 or 18 carbon atoms, and M⁺ being selected from positively charged ions, such as alkali metal ions or positively charged primary, secondary, tertiary or quaternary ammonium ions, preferably M⁺ being a sodium ion.

7. The teichoic acid used according to any one of claims 2 to 5, wherein the lipoteichoic acid is a purified lipoteichoic acid isolatable from bacteria of the genus *streptococcus,* preferably from *streptococcus sp.,* most preferably from *streptococcus sp.* (DSM 8747), the Streptococcus sp. PT strain DSM 8747, preferably containing a beta-galactofuranosyl(1-3)glycerol-di-ester moiety.

8. The teichoic acid used according to claim 7, wherein the lipoteichoic acid is LTA-T, a purified lipoteichoic acid isolatable from the Streptococcus sp. PT strain DSM 8747.

9. The teichoic acid used according to claim 2, wherein the lipoteichoic acid is 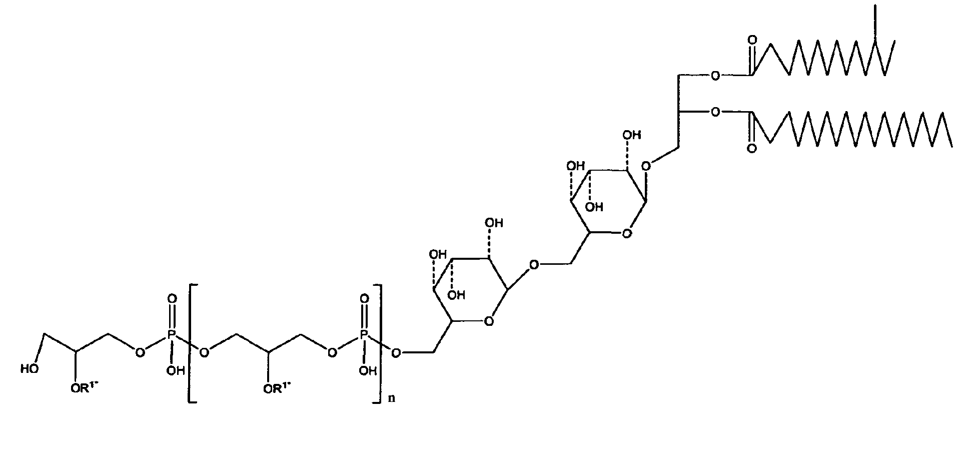 , wherein X is H or D-ala and the mean value of "n" is 16 to 40.

10. The teichoic acid used according to claim 2, wherein the teichoic or lipoteichoic acid is 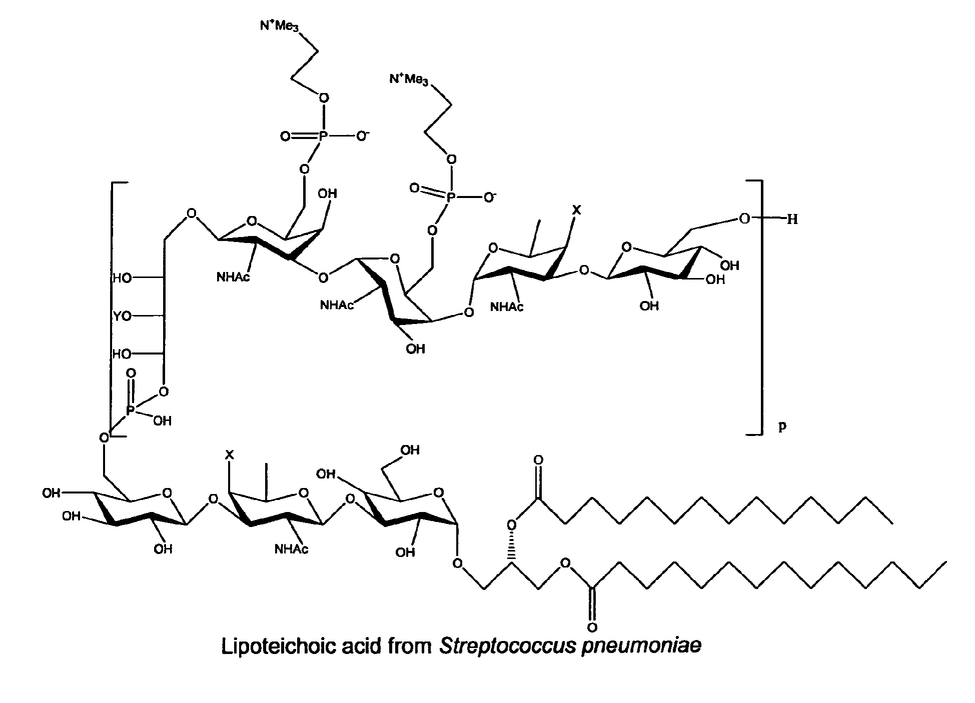 wherein
either X is NH₃⁺, or NHAc; Y is H, D-Ala, or α-GalNAc; and p is up to 8;
or X is NH₃⁺; Y is H; and p is 1.

11. The teichoic acid used according to any one of claims 1 to 10, wherein the teichoic or lipoteichoic acid or its physiological acceptable salt is prepared for the use in the treatment of malignant liquor cerebrospinalis in form of an injectable fluid.

12. The teichoic acid used according to any one of claims 1 to 11, wherein the teichoic or lipoteichoic acid or its physiological acceptable salt is used by injection of the teichoic or lipoteichoic acid into the fluid of the malignant liquor cerebrospinalis;
and/or
wherein the teichoic or lipoteichoic acid or its physiological acceptable salt is used by applying 100 µg to 500 µg, preferably 250µg, of teichoic or lipoteichoic acid or its physiological acceptable salts, preferably by injection of 100 µg to 500 µg, preferably 250 µg, of the teichoic or lipoteichoic acid or its physiological acceptable salts.

13. Use of a teichoic or lipoteichoic acid or its physiological acceptable salts as defined in any of claims 1 to 10 in the manufacture of a medicament for the treatment of malignant liquor cerebrospinalis.

14. A pharmaceutical composition comprising teichoic or lipoteichoic acid or its physiological acceptable salts as defined in any of claims 1 to 10 and optionally at least one physiologically acceptable excipient for use in the treatment of malignant liquor cerebrospinalis.

15. The pharmaceutical composition according to claim 14
being in form of an injectable fluid, preferably comprising injectable liquid carriers, such as water or suitable alcohols, and/or pharmaceutical excipients for injection, such as stabilizing agents, solubilizing agents, and buffers;
and/or
comprising 100 µg to 500 µg, preferably 250 µg of teichoic or lipoteichoic acid or its physiological acceptable salts, preferably being in form of an injectable fluid comprising 100 µg to 500 µg, preferably 250 µg of teichoic or lipoteichoic acid or its physiological acceptable salts, preferably in form of an injectable fluid to be injected by intraperitoneal route.
